# EUROPEAN PATENT SPECIFICATION

(11) **EP 4 380 449 B1**
(45) Date of publication and mention of the grant of the patent: **10.09.2025**
(21) Application number: 22757287.2
(22) Date of filing: 28.07.2022
(51) Int. Cl.: A61B 5/28, A61B 5/282, A61B 5/00

(54) **POSITIONING OF A SENSOR PATCH**
POSITIONIERUNG EINES SENSORPFLASTERS
POSITIONNEMENT D'UN PATCH DE CAPTEUR

(30) Priority: 03.08.2021 EP 21189496; 12.11.2021 EP 21207914
(43) Date of publication of application: 12.06.2024
(73) Proprietor: Koninklijke Philips N.V., 5656 AG Eindhoven (NL)
(72) Inventor: TE VELDE, Mart, 5656 AG Eindhoven (NL)
(74) Representative: Philips Intellectual Property & Standards
(86) International application number: PCT/EP2022/071183
(87) International publication number: WO 2023/012025

(56) References cited:
- EP-A1- 3 936 039
- WO-A1-2017/108215
- WO-A1-2020/179845
- US-A1- 2011 279 963
- US-A1- 2014 062 508

## Description

### FIELD OF THE INVENTION

The invention relates to a system comprising a belt and a sensor patch, and more specifically to the positioning of a sensor patch on a user.

### BACKGROUND OF THE INVENTION

US20140062508A1 discloses a non-resistive contact sensor assembly which includes an electric field sensor device, including a dry electrode component for receiving an electrical signal from an object of interest and a signal processing component for processing the electrical signal, and a casing in which the signal processing component is surrounded or embedded.

WO2017108215A1 discloses a health-monitor patch comprising at least one physiological sensor and digital processor. The patch is configured to be adhered to the skin of a subject.

US20110279963A1 discloses an electronic device for long-term adhesion to a mammal. The device includes a housing with an electronic component.

Wearable devices having electrodes can be used to determine vital signs of a user. One of their uses includes pregnancy monitoring, such as monitoring fetal and maternal heartrate and uterine activity of a pregnant woman. Wet electrodes are typically used for such devices, requiring a gel to be placed between the electrode and the skin of the user, for enhancing skin contact and signal transmission. Wet electrodes are attached to the skin with an adhesive, where the adhesive function is often combined with the gel.

Before attaching the wet electrodes, preparation of the skin of the user is needed. This entails sandpaper-assisted skin abrasion, to ensure good contact and minimize motion and electrostatic artefacts. Because of the required gel, wet electrodes are for one time use only and they are disposed after the measurement.

Relatively recently, wearable devices using dry electrodes have been introduced. Dry electrodes can be re-used, can be cleaned, they have a longer shelf life, and in principle they do not require skin preparation. However, as is also the case with wet electrodes, the measurement is sensitive to motion artefacts.

Another type of re-usable electrodes are semi-dry electrodes. These require only a small amount of electrolyte fluid, stored in a reservoir inside the electrode structure and they can be re-used for a limited number of times, depending on the electrolyte fluid left in their reservoir. Their setup is fast and convenient, similar to that of dry electrodes.

There is currently considerable attention towards the positioning of re-usable electrodes on a user. One major issue that needs to be resolved, is the reduction of motion artefacts caused by displacement of the electrodes with respect to the skin of the user.

### SUMMARY OF THE INVENTION

It is, inter alia, an object of the invention to provide a system comprising a belt and a sensor patch, the sensor patch comprising features, which enable it to minimize its movement with respect to a user's skin, thus leading to the reduction of motion artefacts.

The invention is defined by the independent claim. Advantageous embodiments are defined in the dependent claims.

The invention provides a system comprising a belt and a sensor patch configured to be positioned on a user by means of the belt, wherein the sensor patch is separate from the belt, the sensor patch comprising:
a first side having an electrode configured to contact the user's skin, and
a second side opposed to the first side, the second side having a curved protrusion located opposite to a part on the first side where the electrode is positioned, the sensor patch being configured to allow the belt to slide over the curved protrusion, and to exercise a normal force on the sensor patch mainly at the part where the electrode is positioned to produce a friction force between the electrode and the user's skin.

An advantage thereof is that the belt exercises a normal force on the sensor patch mainly at the part where the electrode is positioned, thereby ensuring contact of the electrode with the user's skin and that friction between the electrode and the user's skin is optimized. Another advantage thereof is that the curved protrusion allows the belt to slide over the curved protrusion, and thus the sensor patch, while the friction force resulting from the normal force ensures that the electrode remains in place, without more than a minimal movement of the sensor patch with respect to the skin. If the sensor patch has multiple electrodes on the first side, preferably the second side has protrusions opposite each of those electrodes.

The sensor patch may be used for example for monitoring physiological parameters of a user, such as electrical activity of the heart, electrical activity of the brain, or pregnancy monitoring e.g. fetal heart rate, maternal heart rate, maternal uterine contractions, maternal respiration rate.

The electrode may be any electrode and preferably a re-usable electrode e.g. a dry electrode or a semi-dry electrode. Using dry or semi-dry electrodes offers advantages over using wet electrodes, such as re-usability, no need for skin preparation and longer shelf life. A further advantage of dry electrodes is increased re-usability in comparison to semi-dry electrodes and their ability to be cleaned.

Usually, sensor patches using wet, but also using other kinds of electrodes (e.g. semi-dry, dry) are attached to the skin using an adhesive. According to the invention, the sensor patch is configured to be positioned on a user by means of a belt. One advantage thereof is that the sensor patch can be cleanable. In contrast, it is very challenging for a sensor patch using adhesives to be cleanable, as water or other cleaning agents may damage the adhesive, even if it is protected by a film. Also, a sensor patch configured to be positioned on a user by means of a belt is more robust and has a longer shelf life than a patch configured to be positioned on a user by means of an adhesive, as the adhesive even if protected by a film, will degrade e.g., due to environmental conditions, aging, etc. Furthermore, the solution using a belt enables reusability and freedom to re-position and slide the patch over the skin, even during operation, without restriction to the number of times this can be done. Such re-positioning or sliding for a sensor patch designed to be positioned using adhesives, is either not possible, or limited.

The location of the sensor patch on the user may depend on the measurements of interest, e.g. for pregnancy monitoring, the sensor patch should preferably be placed on the belly of the user. For positioning the sensor patch on the user, the belt is fastened around the body or body parts of the user, covering fully or partially the sensor patch, e.g. the belt is fastened around the waist of the user, when the sensor patch is placed on the belly, or around the torso when the sensor patch is placed on the user's chest.

In order to reduce the movement of the sensor patch with respect to the skin of the user, the invention aims to decouple the sensor patch from movement of the belt in the plane lateral to the skin of the user, while maintaining the pressing force of the belt perpendicular to the skin. Such movement of the belt can be caused when the user is moving or changing posture. To achieve this goal, the sensor patch may have features elaborated hereinafter.

Such a feature is that the sensor patch is designed to be separate from the belt, thus the electrode used for measuring physiological parameters of a user is not integrated or fastened on the belt by means of e.g. hooks, buttons, or any other fastening systems.

The first side of the sensor patch is configured to contact the user's skin and comprises one or more electrodes. The curved protrusion at the second side of the sensor patch serves the purpose of being the area of contact with the belt. Through the curved protrusion, the belt presses the sensor patch against the skin of the user, ensuring the contact of the electrode with the skin. Furthermore, because the surface of the protrusion is curved, the belt can slide over the sensor patch, while pressing the sensor patch against the skin of the user. Therefore, when the user is moving, the belt can slide over the sensor patch, while the sensor patch and thus the electrode, remains in contact and does not move with respect to the user's skin. As a result, movement artefacts are reduced.

Further advantage of the sensor patch being separate from the belt is that it can be easily cleaned e.g. with a wet cloth, or with alcohol.

The sensor patch may have a plurality of curved protrusions at the second side. The effect is that the plurality of curved protrusions serves as a plurality of areas of contact with the belt, further improving the contact of the sensor patch with the user's skin.

The sensor patch may have a plurality of electrodes at the first side and the second side has a corresponding plurality of curved protrusions respectively located opposite the electrodes. The effect is that the belt can slide over the sensor patch and press the electrodes against the skin of the user, through the curved protrusions. As a result, the contact of the electrodes with respect to the skin of the user is improved, while movement of the electrodes with respect to the skin of the user is reduced.

The sensor patch may comprise a first friction element on the first side. The advantage of the first friction element is the creation of additional friction between the sensor patch and the user's skin.

The sensor patch may comprise a first friction element on the first side located next to the electrode. The advantage of the first friction element is the creation of additional friction between the sensor patch and the area of the skin in contact with the electrode, which hinders the movement of the electrode with respect to the skin.

The sensor patch may have one or more lateral extensions. **In** order to ensure that the lateral extension remains in contact with the skin, the second side of the lateral extension has a curved protrusion, thereby pressing the lateral extension towards the skin.

The lateral extension may comprise a second friction element on the first side, which creates additional friction between the lateral extension and the skin. The additional friction from the second friction element hinders the movement, twisting or curling of the lateral extension, as the belt slides over the curved protrusion of the lateral extension. Twisting or curling of the lateral extension may become relevant in case the sensor patch or parts thereof, e.g. parts of the lateral extension, are made of a flexible material.

The first and second friction elements may be made of an anti-slip material, e.g. plastic, rubber or fabric, which prevents sliding or moving of the sensor patch with respect to the skin. The advantage of using these materials is that they are robust, cleanable, re-usable and have a long shelf-life, thus they contribute to the robustness and re-usability of the sensor patch. Because of these advantages, using adhesive materials instead of first and/or second friction elements is not preferred.

The sensor patch may be flexible, which provides the advantage that it can better follow the curvature of the skin of the user e.g. follow the shape of the belly. The sensor patch or parts thereof may be elastically or plastically deformed.

The curved protrusion may be made of a low-friction material, i.e. with friction characteristics, such that the belt can slide over the sensor patch, without more than a minimal movement of the sensor patch with respect to the skin. The advantage is ensuring that the friction between the belt and the sensor patch is low enough that the sensor patch will not move with respect to the user when the belt slides over the sensor patch. Thus, these friction characteristics of the curved protrusion facilitate the sliding of the belt over the sensor patch.

The curved protrusions may be separate from each other and may be separably or inseparably attached to the first side, wherein the first side is partly or solely made of a flexible material. The advantage of this configuration is that the sensor patch may better follow the body contour, and thus the contact of the electrodes with the skin of the user can be improved.

The sensor patch may comprise one or more motion sensors, like an accelerometer and/or an optical motion sensor. The advantage of an optical motion sensor is that it can directly detect movement and/or speed of the sensor patch with respect to the skin. Thus, its signal may provide input for improving measurements suffering from motion artefacts.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a known sensor patch.
Fig. 2 shows an example of the sensor patch for use in a system according to the invention.
Fig. 3a and 3b show examples of the sensor patch for use in the invention, depicting the forces exerted by a belt to the sensor patch.
Fig. 4a and 4b show another example of the sensor patch for use in the invention.
Fig. 5 shows another example of the sensor patch for use in the invention.
Fig. 6 shows another example of the sensor patch for use in the invention.
Fig. 7 shows another example of the sensor patch for use in the invention.
Fig. 8 shows another example of the sensor patch for use in the invention.
Fig. 8a shows another example of the sensor patch for use in the invention.
Fig. 9 shows an example of a system comprising a sensor patch and a belt according to the invention.
Fig. 10 shows another example of the system comprising the sensor patch and the belt according to the invention.

### DESCRIPTION OF EMBODIMENTS

The invention will be described with reference to the Figures. The detailed description and specific examples, while indicating exemplary embodiments of the devices and methods, are intended for purposes of illustration only and are not intended to limit the scope of the invention. These and other features, aspects, and advantages of the device and methods of the present invention will become better understood from the following description, appended claims, and accompanying drawings. The Figures are merely schematic and are not drawn to scale. It should also be understood that the same reference numerals are used throughout the Figures to indicate the same or similar parts.

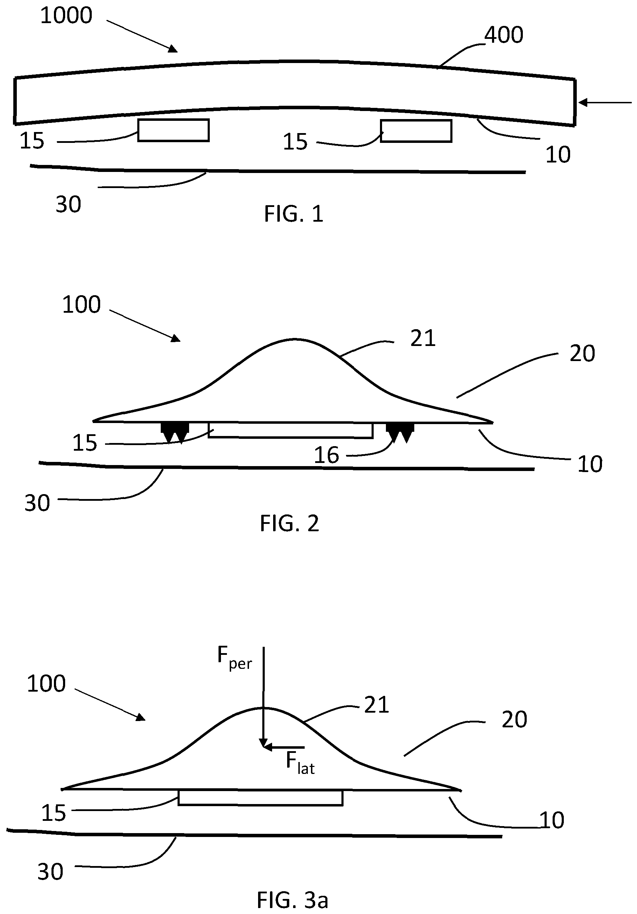

The invention provides a sensor patch for monitoring physiological parameters of a user, using an electrode. The sensor patch is configured to be positioned on a user by means of a belt. A known solution is to fasten an electrode on a belt and Fig. 1 shows an example of how to implement such a known arrangement.

Fig. 1 shows a known sensor patch 1000 where one or more electrodes 15 are either integrated or hooked on a belt 400. The electrode 15 is located at a first side 10 of the sensor patch 1000 configured to contact the user's skin 30. A disadvantage of such an arrangement is that in case the belt 400 moves or rotates, as depicted in Fig. 1 with an arrow, the electrode 15 which is fastened on the belt 400 will also move or rotate with respect to the skin 30. Such a movement results to undesirable motion artefacts. Furthermore, in such an arrangement where the electrode 15 is fastened to the belt 400, a good fit of the belt 400 with the body would be needed, in order to ensure that the electrode is placed on a suitable position on the user's body. Thus, multiple belt shapes or sizes would be needed to fit different users, e.g. different belts would be needed for pregnancy monitoring depending on the belly-shapes of a wide range of pregnant women.

Fig. 2 shows an example of the sensor patch 100 for use in the invention. Sensor patch 100 comprises a first side 10 and a second side 20. The first side 10 faces the skin of a user. An electrode 15 is placed at first side 10 and the electrode 15 is configured to contact the user's skin 30. The electrode 15 may be a re-usable electrode. The sensor patch 100 or parts thereof may be flexible. It may be made of any suitable material, e.g. a polymer or metal, and it may have sufficient flexibility so as to enable conformal application to the surface of the skin 30.

The second side 20 is opposed to the first side 10. The sensor patch 100 is positioned on the skin 30 of the user with the help of a separate belt (not shown in Fig. 2). The belt provides the necessary force perpendicular Fₚₑᵣ to the skin 30 of the user as shown in Fig. 3a. The force Fₚₑᵣ ensures good contact of the electrode 15 with the skin 30 of the user and it creates friction between the sensor patch 100 and the skin 30 of the user, which friction hinders movement of the sensor patch 100 with respect to the skin 30.

The belt may also exert a lateral force to the sensor patch 100, which is parallel to the plane of the skin 30 of the user Fₗₐₜ as shown in Fig. 3a. This force appears when the user moves or changes posture, due to the fact that the belt is fastened to the body or parts of the body of the user and therefore adapts to the user's motion. Force Fₗₐₜ tends to move the sensor patch with respect to the skin. In order to reduce Fₗₐₜ, to the point that the sensor patch will not move with respect to the skin 30 of the user, the belt needs to slide over the second side 20, effectively decoupling the force that the belt exerts on the sensor patch 100 in the plane parallel to skin 30 from the sensor patch 100.

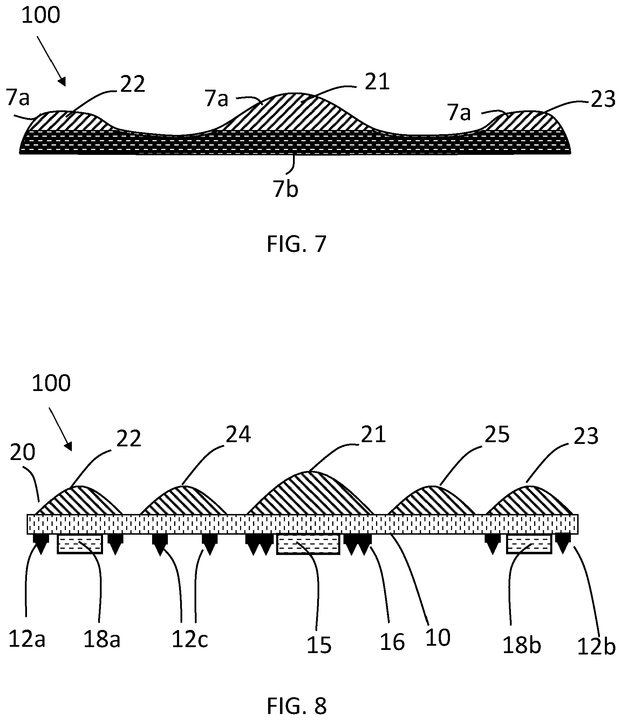

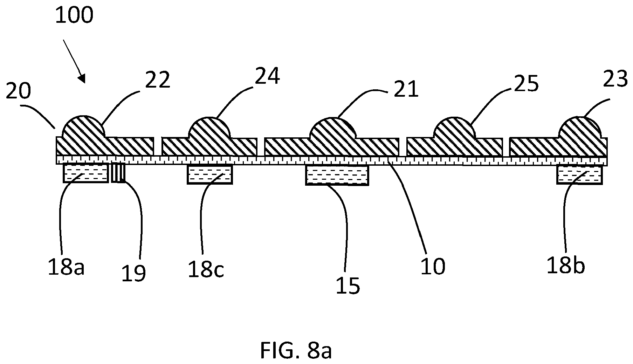

For this reason, the second side 20 has a curved protrusion 21, which is located opposite to a part on the first side 10 where the electrode 15 is positioned. The curved protrusion 21 is schematically shown as an integrated part of the sensor patch 100 in Fig. 2, nevertheless it is to be understood that the curved protrusion may also be separable from the sensor patch 100, as exemplary shown in Fig. 8. The curved protrusion comprises the part of the sensor patch 100 which is configured to be in contact with the belt. As the curved protrusion 21 on the second side 20 is located on top of electrode 15, the force perpendicular Fₚₑᵣ to the skin 30 of the user exerted from the belt to the curved protrusion 21, extends to the electrode 15.

The curved protrusion 21- 25 is preferably smooth, preferably having an even and regular surface, preferably free from perceptible sharp ends. The smooth surface reduces friction between the belt and the curved protrusion 21 - 25, thus facilitating the movement of the belt with respect to the sensor patch 100.

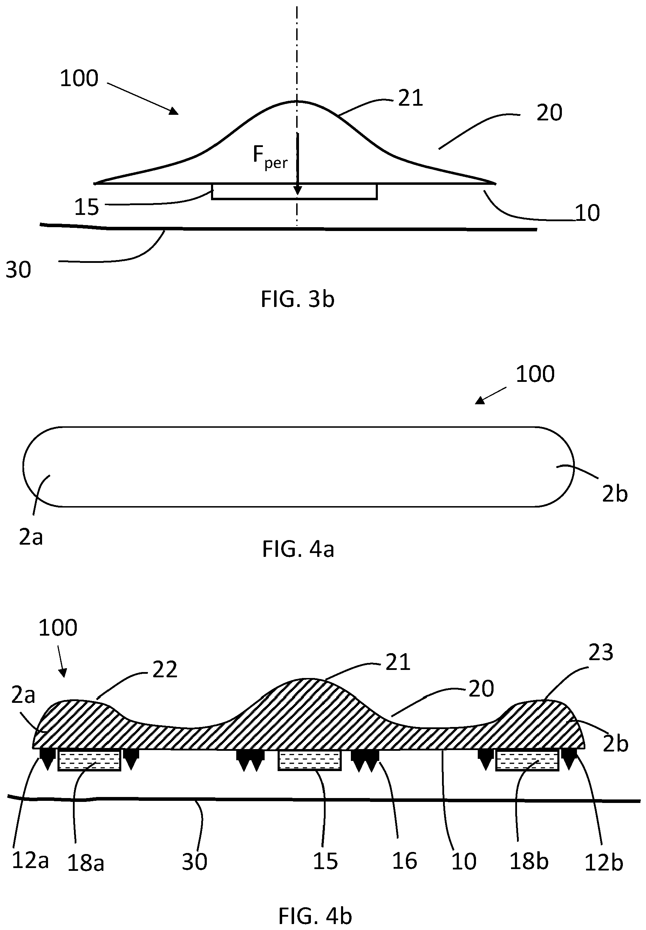

In a non-limiting example shown in Fig. 3b, the axis of the curved protrusion 21 perpendicular to the skin of the user which passes through the centroid of the curved protrusion, substantially coincides to the axis of the electrode 15 perpendicular to the skin of the user which passes through the centroid of the electrode 15. With such an arrangement, the force perpendicular Fₚₑᵣ to the skin 30 of the user is exerted to the centroid of the electrode 15. As a result, an even pressure distribution along the radius of the electrode 15 is achieved and any torque about the center of mass of the electrode 15 is minimized, thus the contact of the electrode 15 with the skin of the user is improved.

Turning back to Fig. 2, the sensor patch 100 may comprise a first friction element 16, located on the first side 10, which increases friction between the sensor patch 100 and the skin 30 of the user. The friction element 16 may be located next to the electrode 15. The first friction element 16 is made of an anti-slip material, e.g. plastic, rubber or fabric, which prevents sliding or moving of the sensor patch 100, with respect to the skin 30. In the example shown in Fig. 2, the first friction element 16 surrounds the electrode 15, but in other examples the first friction element 16 may not surround the electrode 15.

The curved protrusion 21 - 25 of the sensor patch 100 may partly or completely be made of a low-friction material, i.e. with friction characteristics allowing the belt to slide over the sensor patch 100, without causing more than a minimal movement of the sensor patch 100 with respect to the skin 30. Examples of such low-friction materials are PTFE, Polyether ether ketone, Polyphenylene sulfide, Nylon, Polyoxymethylene, or Polyester. Examples of desired friction characteristics are materials with static friction coefficient less than 0.5, preferably less than 0.3, and more preferably less than 0.1. The above examples of materials or materials having desired friction characteristics are considered in practical circumstances as low-friction materials. Although the belt may be able to slide over the sensor patch 100 due to the curved protrusion 21 - 25 using such low-friction materials may facilitate the sliding of the belt over the sensor patch 100. It is to be understood that the sensor patch may comprise such materials only at the curved protrusion 21 - 25 or at part of the curved protrusion 21 - 25, while the rest of the sensor patch 100 may be made of different materials.

The curved protrusion 21-25 of the sensor patch 100 may partly or fully made of hard plastic. Examples of hard plastics are HDPE, UHMW, PVC or PP. Preferably, the static friction coefficient of the curved protrusion 21-25 is less than 0.5, preferably less than 0.3, and more preferably less than 0.1.

The sensor patch 100 may also comprise a processor, for processing the signals received from the electrode 15, a memory module, for storing raw or processed data, a communication module, for transmission or reception of data over a wire-based or wireless based communication technology and a power module, for supplying with power the modules of the sensor patch 100. It is to be understood that the sensor patch may also comprise other modules, assisting the operation of the sensor patch 100, for monitoring physiological parameters of a user.

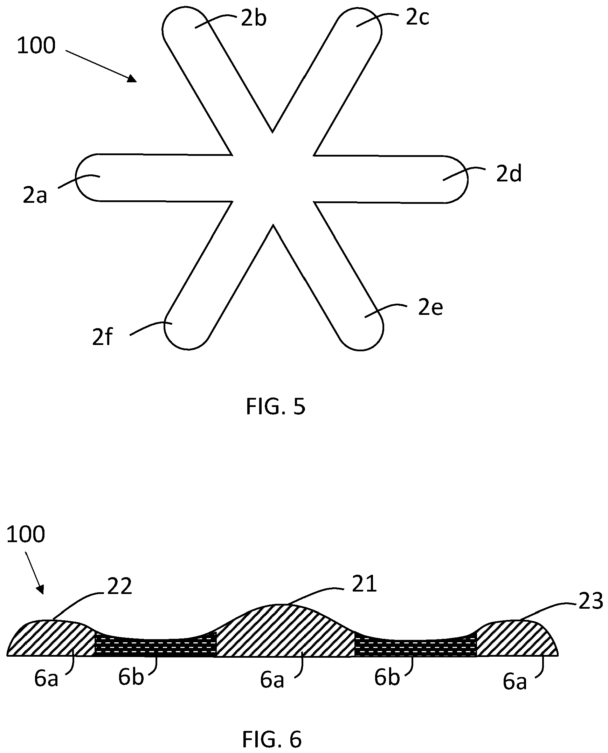

In a further embodiment, the sensor patch 100 may have a lateral extension. Fig. 4a shows a top view and Fig. 4b a cross section of the sensor patch 100 having two lateral extensions 2a and 2b. Fig. 5 shows a top view of another embodiment of the sensor patch having 6 lateral extensions. Figs. 4a, 4b and 5 merely depict two examples of a sensor patch 100 having lateral extensions, but these are non-limiting examples, and more shapes and layouts can be used.

In the example shown in Fig. 4b, electrode 18a is placed at the first side 10 of lateral extension 2a, surrounded by an optional friction element 12a, and electrode 18b is placed at the first side 10 of lateral extension 2b, surrounded by an optional friction element 12b. In other examples, the friction elements 12a, 12b may not (completely) surround the corresponding electrodes 18a, 18b, or an electrode 18 may be placed at the first side 10 without a friction element 12.

Electrodes 15, 18a and 18b may all be of the same type, or may all be different, depending on the intendent use and measurements, and may preferably be medical electrodes. The electrodes may be silver chloride electrodes.

The cross section of Fig. 4b shows that the second side 20 comprises curved protrusions 21, 22 and 23. Curved protrusion 21 corresponds to the area of the contact of the belt with the mid-section of the sensor patch 100, curved protrusion 22 corresponds to the area of contact of the belt with lateral extension 2a, and curved protrusion 23 corresponds to the area of contact of the belt with lateral extension 2b.

The friction elements 12a, 12b may have the same or different shape, and may be made of the same or different material as the first friction element 16 e.g. plastic, rubber or fabric, which prevents sliding or moving of the sensor patch 100, with respect to the skin of the user 30. A friction element at a lateral extension of the sensor patch 100 hinders the relative movement of the lateral extension with respect to the skin and possibly, the movement or rotation of the whole sensor patch 100, thus the sensor patch remains in place while the belt can slide over the sensor patch (e.g. caused by movements of the patient).

It is to be understood that the sensor patch 100 may be made of more than one material. For example, the material of the sensor patch connecting the curved protrusions 21, 22, 23, which are configured to be in contact with the belt, may be made of a flexible material, such as flexible laminates, or elastomeric materials. Fig. 6 shows an example where the curved protrusions 21, 22 and 23 are made of material 6a, e.g. a low-friction material and the material of the sensor patch 100 connecting the curved protrusions is made of material 6b, e.g. a flexible material.

Fig. 7 shows another example, where part of the curved protrusions 21, 22 and 23 configured to be in contact with the belt is made of material 7a, e.g. a low-friction material, and the rest of the sensor patch 100, may be made of material 7b, e.g. a flexible material, e.g. flexible laminates, elastomeric materials, and it may also be a flexible electronic circuit, integrating wiring or other electronic components in a flexible substrate.

Fig. 8 shows an example, where the curved protrusions 21, 22, 23, 24 and 25 may be separable from the sensor patch 100.

In another example shown in Fig. 8, friction element 12c located on the first side 10 is not placed next to an electrode. Its purpose is to hinder the movement of the first side 10 with respect to the skin.

In yet another example shown in Fig. 8, no electrodes are positioned opposite to a part of the second side 20, where curved protrusions 24 and 25 are located. The purpose of curved protrusions 24 and 25 is to increase the areas of contact of the sensor patch 100 with the belt, thereby further improving the contact of the sensor patch 100 with the user's skin and further hindering the movement, twisting or curling of the sensor patch 100.

Fig. 8a shows an example, where the curved protrusions 21, 22, 23, 24 and 25 are not directly connected to each other, i.e. they are separate from each other. Thus, the curved protrusions 21, 22, 23, 24 and 25 are segments comprising the second side 20. The curved protrusions are separably or inseparably attached to the first side 10 and may be made of hard plastic or any other rigid material, e.g. metal. Preferably, the static friction coefficient of the curved protrusions is less than 0.5, preferably less than 0.3, and more preferably less than 0.1. The first side 10 is partly or solely made of a flexible material, e.g. flexible laminates, or elastomeric materials and/or it may be a flexible electronic circuit, integrating wiring or other electronic components in a flexible substrate.

The result of the curved protrusions being segments of the second side 20 and being attached to a flexible side 10, is that the flexible backing of the first side 10 acts as a hinge between the segments of the second side 20, i.e. the protrusions. The advantage of this configuration is that the sensor patch 100 can better follow the body contour, thus improving the contact of the electrodes 15, 18a, 18b, 18c with the skin of the user.

In another example shown in Fig. 8a, the sensor patch 100 comprises a motion sensor 19, e.g. an accelerometer, a gyroscope, a magnetometer, or an optical motion sensor (like the one used in a computer mouse). While the sensor patch 100 is designed to minimize its movement with respect to the skin of the user, nevertheless, such a movement may still occur. For this reason, detecting movement of the sensor patch with respect to the skin of the user with the help of a motion sensor may provide input for improving measurements suffering from motion artefacts.

Accelerometers, gyroscopes and magnetometers detect movement in general, i.e. movement of the user, even if the sensor patch does not move with respect to the skin, thus the detection and/or prediction of motion artefacts can be achieved indirectly, by processing the motion sensor signals using appropriate algorithms. On the other hand, an optical motion sensor has the advantage that it can directly detect movement and/or speed of the sensor patch with respect to the skin. In one example, only an accelerometer, gyroscope or magnetometer may be used. In another example only an optical motion sensor may be used. In yet another example, an optical motion sensor may be used in conjunction with an accelerometer, gyroscope or magnetometer, providing extra input to the measurements of the accelerometer, gyroscope or magnetometer, and thus further improving the prediction and or detection of the motion artefacts.

In the example shown in Fig. 8a, only one motion sensor 19 is depicted. Nevertheless, more than one motion sensors may be used, preferably placed near an electrode.

In an embodiment, the sensor patch is safe to use in a magnetic resonance environment. The sensor patch may for example fulfil the recommendations for an MR Conditional device, of the US Food and Drug Administration: Testing and Labeling Medical Devices for Safety in the Magnetic Resonance (MR) Environment, May 20, 2021.

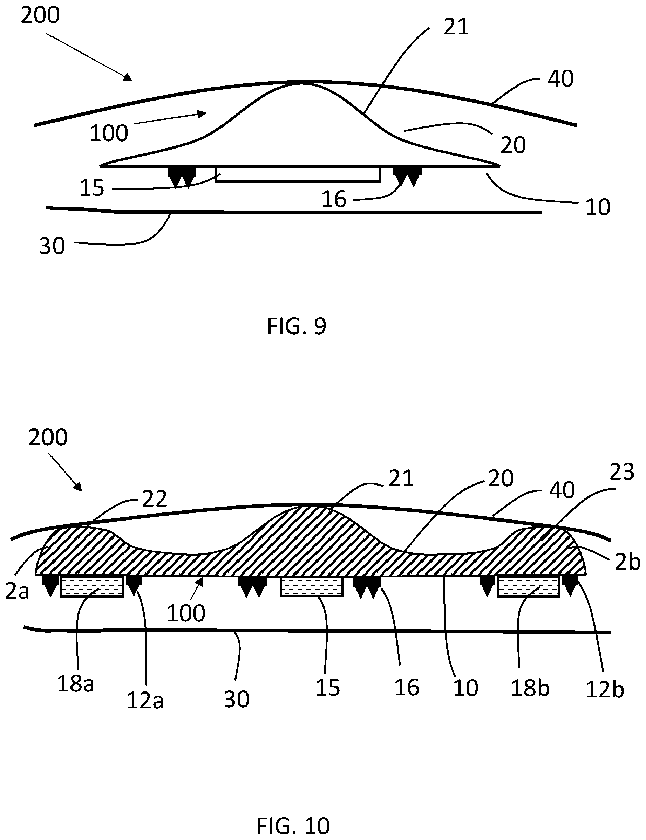

In a second aspect of the invention, a system 200 comprises a belt 40, and a sensor patch as already described herein. Fig. 9 and Fig. 10 show such examples, wherein the belt 40 contacts the sensor patch 100 at the curved protrusion 21 of the second side 20.

The belt may be made of a stretchable fabric, ensuring a good fit with the body, irrespective of the shape and the size of the body, e.g. one belt size may be used for pregnancy monitoring, independently of the belly shape or size of a wide range of pregnant women. Furthermore, any belt may be used to position a sensor patch on a user, with size or stretchability suitable to be fastened around the user's body or the user's body parts of interest. The belt may also be washable e.g. in a washing machine, so it can be cleaned before being re-used.

In summary, the invention provides a system 200 comprising a belt 40 and a sensor patch 100 configured to be positioned on a user by means of a belt, wherein the sensor patch comprises a first side 10 having an electrode 15 configured to contact the user's skin 30 and a second side 20 opposed to the first side, the second side having a curved protrusion 21 located opposite to a part on the first side 10 where the electrode 15 is positioned. In order to minimize the movement of the sensor patch with respect of the skin of the user, the invention aims to decouple the sensor patch from movement of the belt in the plane lateral to the skin, while maintaining the pressing force of the belt perpendicular to the skin of the user. A system 200 comprising the sensor patch and the belt is also provided.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements.

## Claims

1. A system (200) comprising:
a belt (40), and,
a sensor patch (100) configured to be positioned on a user by means of the belt, wherein the sensor patch (100) is separate from the belt, the sensor patch comprising:
a first side (10) having an electrode (15) configured to contact the user's skin (30), and
a second side (20) opposed to the first side, the second side having a curved protrusion (21) located opposite to a part on the first side (10) where the electrode (15) is positioned, the sensor patch (100) being configured to allow the belt
to slide over the curved protrusion (21), and
to exercise a normal force on the sensor patch (100) mainly at the part where the electrode is positioned to produce a friction force between the electrode (15) and the user's skin (30).

2. The system (200) as claimed in claim 1, wherein the second side (20) has a plurality of curved protrusions (21, 22, 23, 24, 25).

3. The system (200) as claimed in claim 1, wherein the first side (10) has a plurality of electrodes (15, 18a, 18b, 18c), and the second side (20) has a corresponding plurality of curved protrusions (21, 22, 23, 24) respectively located opposite the electrodes (15, 18a, 18b, 18c).

4. The system (200) as claimed in any of the preceding claims, wherein:
the sensor patch comprises a first friction element (16) on the first side (10).

5. The system (200) as claimed in claim 4, wherein:
the first friction element (16) on the first side (10) is located next to the electrode (15).

6. The system (200) as claimed in any of the preceding claims, wherein:
the sensor patch has a lateral extension (2a, 2b) provided with a further electrode (18a, 18b), wherein the second side (20) of the lateral extension (2a, 2b) has a curved protrusion (22, 23) opposite the further electrode (18a, 18b).

7. The system (200) as claimed in claim 6, wherein:
the lateral extension (2a, 2b) comprises a second friction (12a, 12b) element on the first side (10).

8. The system (200) as claimed in any of the preceding claims, wherein:
the sensor patch is flexible.

9. The system (200) as claimed in any of the preceding claims, wherein:
the curved protrusion (21, 22, 23, 24, 25) comprises a low-friction material.

10. The system (200) as claimed in any of claims 1-9, wherein:
the second side (20) has a plurality of curved protrusions (21, 22, 23, 24, 25) that are separate from each other, and are separably or inseparably attached to the first side (10), and wherein:
the first side (10) is partly or solely made of a flexible material.

11. The system (200) as claimed in any of the preceding claims, wherein:
the sensor patch comprises a motion sensor, preferably an optical motion sensor (19).

## Patentansprüche

1. System (200), umfassend:
einen Gurt (40), und
ein Sensorpflaster (100), das so konfiguriert ist, dass es mittels des Gurtes an einem Benutzer positioniert werden kann, wobei das Sensorpflaster (100) vom Gurt getrennt ist, wobei das Sensorpflaster umfasst:
eine erste Seite (10), die eine Elektrode (15) aufweist, die so konfiguriert ist, dass sie die Haut (30) des Benutzers berührt, und
eine zweite Seite (20), die der ersten Seite gegenüberliegt, wobei die zweite Seite einen gekrümmten Vorsprung (21) aufweist, der sich einem Teil auf der ersten Seite (10), an dem die Elektrode (15) positioniert ist, gegenüberliegend befindet, wobei das Sensorpflaster (100) so konfiguriert ist, dass es dem Gurt ermöglicht,
über den gekrümmten Vorsprung (21) zu gleiten, und
eine Normalkraft auf das Sensorpflaster (100) hauptsächlich an dem Teil, an dem die Elektrode positioniert ist, auszuüben, um eine Reibungskraft zwischen der Elektrode (15) und der Haut (30) des Benutzers zu erzeugen.

2. System (200) nach Anspruch 1, wobei die zweite Seite (20) eine Vielzahl von gekrümmten Vorsprüngen (21, 22, 23, 24, 25) aufweist.

3. System (200) nach Anspruch 1, wobei die erste Seite (10) eine Vielzahl von Elektroden (15, 18a, 18b, 18c) aufweist und die zweite Seite (20) eine entsprechende Vielzahl von gekrümmten Vorsprüngen (21, 22, 23, 24) aufweist, die sich jeweils den Elektroden (15, 18a, 18b, 18c) gegenüberliegend befinden.

4. System (200) nach einem der vorstehenden Ansprüche, wobei:
das Sensorpflaster ein erstes Reibungselement (16) auf der ersten Seite (10) umfasst.

5. System (200) nach Anspruch 4, wobei:
das erste Reibungselement (16) sich auf der ersten Seite (10) neben der Elektrode (15) befindet.

6. System (200) nach einem der vorstehenden Ansprüche, wobei:
das Sensorpflaster eine seitliche Erstreckung (2a, 2b) aufweist, die mit einer weiteren Elektrode (18a, 18b) versehen ist, wobei die zweite Seite (20) der seitlichen Erstreckung (2a, 2b) einen gekrümmten Vorsprung (22, 23) aufweist, der der weiteren Elektrode (18a, 18b) gegenüberliegt.

7. System (200) nach Anspruch 6, wobei:
die seitliche Erstreckung (2a, 2b) ein zweites Reibungselement (12a, 12b) auf der ersten Seite (10) umfasst.

8. System (200) nach einem der vorstehenden Ansprüche, wobei:
das Sensorpflaster flexibel ist.

9. System (200) nach einem der vorstehenden Ansprüche, wobei:
der gekrümmte Vorsprung (21, 22, 23, 24, 25) ein reibungsarmes Material umfasst.

10. System (200) nach einem der Ansprüche 1-9, wobei:
die zweite Seite (20) eine Vielzahl von gekrümmten Vorsprüngen (21, 22, 23, 24, 25) aufweist, die voneinander getrennt sind und trennbar oder untrennbar an der ersten Seite (10) angebracht sind, und wobei:
die erste Seite (10) teilweise oder ausschließlich aus einem flexiblen Material besteht.

11. System (200) nach einem der vorstehenden Ansprüche, wobei:
das Sensorpflaster einen Bewegungssensor, vorzugsweise einen optischen Bewegungssensor (19), umfasst.

## Revendications

1. Système (200) comprenant :
une ceinture (40), et,
un patch capteur (100) configuré pour être positionné sur un utilisateur au moyen de la ceinture, dans lequel le patch capteur (100) est séparé de la ceinture, le patch capteur comprenant :
un premier côté (10) présentant une électrode (15) configurée pour venir en contact avec la peau (30) de l'utilisateur, et
un second côté (20) opposé au premier côté, le second côté présentant une saillie incurvée (21) située à l'opposé d'une partie du premier côté (10) où l'électrode (15) est positionnée, le patch capteur (100) étant configuré pour permettre à la ceinture
de glisser sur la saillie incurvée (21), et
d'exercer une force normale sur le patch capteur (100) principalement au niveau de la partie où l'électrode est positionnée pour produire une force de frottement entre l'électrode (15) et la peau (30) de l'utilisateur.

2. Système (200) selon la revendication 1, dans lequel le second côté (20) présente une pluralité de saillies incurvées (21, 22, 23, 24, 25).

3. Système (200) selon la revendication 1, dans lequel le premier côté (10) présente une pluralité d'électrodes (15, 18a, 18b, 18c), et le second côté (20) présente une pluralité correspondante de saillies incurvées (21, 22, 23, 24) situées respectivement opposées aux électrodes (15, 18a, 18b, 18c).

4. Système (200) selon l'une quelconque des revendications précédentes, dans lequel :
le patch capteur comprend un premier élément de friction (16) sur le premier côté (10).

5. Système (200) selon la revendication 4, dans lequel :
le premier élément de friction (16) sur le premier côté (10) est situé à côté de l'électrode (15).

6. Système (200) selon l'une quelconque des revendications précédentes, dans lequel :
le patch capteur présente une extension latérale (2a, 2b) munie d'une électrode supplémentaire (18a, 18b), dans lequel le second côté (20) de l'extension latérale (2a, 2b) présente une saillie incurvée (22, 23) opposée à l'électrode supplémentaire (18a, 18b).

7. Système (200) selon la revendication 6, dans lequel :
l'extension latérale (2a, 2b) comprend un second élément de friction (12a, 12b) sur le premier côté (10).

8. Système (200) selon l'une quelconque des revendications précédentes, dans lequel :
le patch capteur est flexible.

9. Système (200) selon l'une quelconque des revendications précédentes, dans lequel :
la saillie incurvée (21, 22, 23, 24, 25) comprend un matériau à faible friction.

10. Système (200) selon l'une quelconque des revendications 1-9, dans lequel :
le second côté (20) présente une pluralité de saillies incurvées (21, 22, 23, 24, 25) qui sont séparées les unes des autres et sont fixées de manière séparable ou inséparable au premier côté (10), et dans lequel :
le premier côté (10) est en partie ou uniquement constitué d'un matériau flexible.

11. Système (200) selon l'une quelconque des revendications précédentes, dans lequel :
le patch capteur comprend un capteur de mouvement, de préférence un capteur de mouvement optique (19).
